# FASCICULE DE BREVET EUROPEEN

(11) **EP 1 543 864 B1**
(45) Date de publication et mention de la délivrance du brevet: **07.03.2007**
(21) Numéro de dépôt: 04292981.0
(22) Date de dépôt: 14.12.2004
(51) Int. Cl.: A61N 1/365, A61N 1/368

(54) **Dispositif médical implantable actif multisite comprenant un mode de resynchronisation des ventricules**
Implantierbares aktives medizinisches Gerät mit Ventrikel-Resynchronisationsmodus
Multisite implantable active medical device comprising a resynchronisation mode of the ventricles

(30) Priorité: 15.12.2003 FR 0314657
(43) Date de publication de la demande: 22.06.2005
(73) Titulaire: ELA MEDICAL, 92541 Montrouge (FR)
(72) Inventeur: Henry, Christine, 75014 Paris (FR); Limousin, Marcel, 75014 Paris (FR)
(74) Mandataire: Dupuis-Latour, Dominique

(56) Documents cités:
- EP-A- 0 488 904
- US-A1- 2003 199 934
- US-B1- 6 181 968
- US-B1- 6 343 231

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne plus particulièrement les prothèses dites "multisite" dans lesquelles des électrodes sont placées en une pluralité de sites respectifs distincts comportant les deux sites ventriculaires, droit et gauche, et au moins un site auriculaire. Il peut s'agir d'une prothèse de type "triple chambre" (double stimulation ventriculaire et détection/stimulation auriculaire droite) ou "quadruple chambre" (double stimulation ventriculaire et double détection/stimulation auriculaire).

La plupart des patients recevant un tel dispositif multisite implanté sont des patients qui présentent une conduction auriculo-ventriculaire habituellement normale (chaque évènement auriculaire est suivi d'une dépolarisation ventriculaire associée) et qui n'ont donc pas d'indication standard pour la pose d'un stimulateur implanté.

Le dispositif multisite est alors indiqué pour traiter l'insuffisance cardiaque de manière à améliorer l'état hémodynamique général de ces patients, par stimulation conjointe et permanente des ventricules gauche et droit afin de resynchroniser ces derniers. Cette thérapie a permis d'observer des résultats souvent spectaculaires pour des patients en insuffisance cardiaque en classe III non améliorée par les traitements classiques.

Ces dispositifs peuvent opérer soit en stimulation synchrone, c'est-à-dire que les deux sites ventriculaires de stimulation reçoivent au même moment l'impulsion de dépolarisation (délai interventriculaire nul), soit, comme décrit dans le EP-A1 108 446 (ELA Médical), avec entre les deux stimulations un délai interventriculaire ajusté de manière à resynchroniser la contraction des ventricules avec une optimisation fine de l'état hémodynamique du patient.

Le point de départ de l'invention résulte d'observations cliniques effectuées sur des patients ayant reçu un tel dispositif lorsqu'apparaît une perte de capture sur l'une des deux sondes ventriculaires.

La perte de capture est une situation dans laquelle l'impulsion de stimulation appliquée à l'électrode de la sonde présente une amplitude insuffisante pour provoquer la dépolarisation du ventricule. Une telle perte de capture est souvent consécutive à une augmentation naturelle, sur le long terme, du seuil d'entraînement.

Du fait de cette perte de capture, la stimulation de l'un des ventricules sera sans effet, et le patient se retrouvera avec une stimulation d'un seul des deux ventricules, droit ou gauche. Cette stimulation d'un seul ventricule peut s'avérer extrêmement délétère si elle se prolonge, car elle peut entraîner une augmentation de la désynchronisation interventriculaire, allant donc à l'inverse du but recherché.

Les inventeurs ont constaté que, dans une telle situation, laisser s'exprimer le rythme spontané du patient peut s'avérer une alternative moins lourde de conséquences que poursuivre une stimulation d'un seul des ventricules.

Essentiellement, l'invention propose, en cas de perte de capture avérée sur l'une des sondes ventriculaires, de ne pas maintenir le mode multisite, mais de commuter le dispositif en mode AAI (stimulation à la demande avec surveillance de l'activité ventriculaire), avec éventuellement commutation ultérieure du mode AAI vers un mode DDD (stimulation double chambre avec association auriculo-ventriculaire) en cas de détection d'un bloc auriculo-ventriculaire (BAV).

Plus précisément, l'invention propose un dispositif multisite apte à fonctionner en mode de resynchronisation ventriculaire du type général divulgué par le EP-A-1 108 446 précité, c'est-à-dire un dispositif multisite triple ou quadruple chambre, comprenant : des moyens de détection ventriculaire droite et gauche, aptes à détecter un potentiel de dépolarisation spontanée dans le ventricule droit et dans le ventricule gauche ; des moyens de détection auriculaire droite, et éventuellement de détection auriculaire gauche, aptes à détecter un potentiel de dépolarisation spontanée dans l'oreillette droite et, éventuellement, dans l'oreillette gauche ; des moyens de stimulation ventriculaire droite et gauche, aptes à délivrer au ventricule droit et au ventricule gauche des impulsions de stimulation respectives ; et des moyens aptes à faire fonctionner le dispositif en mode de resynchronisation des contractions ventriculaires, comprenant des moyens pour établir un délai interventriculaire ajustable entre les instants d'application des impulsions respectivement délivrées au ventricule droit et au ventricule gauche au cours d'un même cycle cardiaque.

Selon l'invention, le dispositif comprend en outre : des moyens de stimulation auriculaire, aptes à délivrer des impulsions de stimulation à l'oreillette droite ; des moyens aptes à faire fonctionner le dispositif en mode AAI avec stimulation auriculaire droite et détection ventriculaire droite ; des moyens de détection de capture ventriculaire, pour déterminer la détection ou la perte de capture après une stimulation ventriculaire droite et après une stimulation ventriculaire gauche ; et des moyens de commutation de mode, aptes à commander un basculement de mode, du mode de resynchronisation vers le mode AAI, en réponse à la détection d'une perte de capture après une stimulation ventriculaire droite ou après une stimulation ventriculaire gauche.

Selon diverses caractéristiques subsidiaires avantageuses :
- le dispositif comprend en outre des moyens d'analyse du rythme cardiaque, aptes à détecter la survenue d'un bloc auriculo-ventriculaire, et des moyens aptes à faire fonctionner le dispositif en mode DDD de stimulation double chambre avec association auriculo-ventriculaire, les moyens de commutation de mode étant également aptes à commander le basculement du mode AAI vers le mode DDD en cas de détection d'un bloc auriculo-ventriculaire ;
- les moyens de commutation de mode sont également aptes à commander le basculement inverse du mode AAI vers le mode de resynchronisation en réponse à la détection d'un rétablissement de la capture ventriculaire droite et gauche ;
- les moyens de commutation de mode comprennent des moyens pour appliquer un délai prédéterminé avant ledit basculement inverse du mode AAI vers le mode de resynchronisation, après ladite détection d'un rétablissement de la capture ventriculaire droite et gauche ;
- les moyens de commutation de mode comprennent des moyens pour, après basculement inverse du mode AAI vers le mode de resynchronisation, inhiber tout nouveau basculement ultérieur du mode de resynchronisation vers le mode AAI pendant une durée prédéterminée.

On va maintenant décrire un exemple de réalisation du dispositif de l'invention, qui peut être mise en oeuvre par une programmation appropriée du logiciel de commande d'un stimulateur connu de type multisite, triple ou quadruple chambre intégrant un mode de resynchronisation des ventricules, un mode AAI avec surveillance de l'activité ventriculaire et un mode DDD de stimulation double chambre avec association auriculo-ventriculaire

L'invention peut notamment être appliquée à des appareils tels que les dispositifs implantés commercialisés par ELA Médical, Montrouge, France. Il s'agit de dispositifs à microprocesseur programmables comportant des circuits pour recevoir, mettre en forme et traiter des signaux électriques recueillis par des électrodes implantées. Il est possible d'y transmettre par télémétrie des logiciels qui seront implantés en mémoire et exécutés pour mettre en oeuvre les fonctions de l'invention décrites ci-dessous. L'adaptation de ces appareils à la mise en oeuvre des fonctions de l'invention est à la portée de l'homme du métier, et elle ne sera pas décrite en détail.

Au départ, le fonctionnement du dispositif est un fonctionnement en mode de resynchronisation ventriculaire, c'est-à-dire que les deux cavités ventriculaires, droite et gauche, sont stimulées conjointement avec un délai VV ajusté, typiquement entre -60 et +60 ms, à intervalles périodiques en fonction de paramètres hémodynamiques mesurés en externe (par exemple par échographie) ou en continu par un capteur implanté (capteur d'accélération endocavitaire, capteur de bioimpédance intracardiaque, etc.). Ces techniques d'analyse de l'état hémodynamique du patient et d'ajustement des paramètres de fonctionnement d'un dispositif asservi sont en elles-mêmes connues et ne seront pas décrites plus en détail. On pourra se référer notamment, outre le EP-A-1 108 446 précité, au EP-A-1 116 497 qui expose la manière de recueillir un signal de bioimpédance intracardiaque pour faire varier le délai interventriculaire d'application des impulsions de stimulation respectives des ventricules droit et gauche dans le sens de l'amélioration du débit cardiaque.

Le dispositif comporte en outre des moyens pour détecter d'éventuelles pertes de capture sur l'une des sondes ventriculaires.

Les algorithmes de détection de capture sont en eux-mêmes bien connus, et l'on pourra se référer au EP-A-0 935 979 ou EP-A-1 287 849, ce dernier document décrivant notamment un algorithme de détection de capture "cycle à cycle", c'est-à-dire dans lequel le dispositif examine à chaque cycle cardiaque - et non plus à intervalles périodiques - si la stimulation a été efficace ou non.

De façon caractéristique de l'invention, en cas de perte de capture avérée sur l'une des sondes ventriculaires l'algorithme commute automatiquement le dispositif du mode initial de fonctionnement en resynchronisation ventriculaire vers un mode de fonctionnement de type AAI, c'est-à-dire avec une stimulation auriculaire simple chambre et une surveillance (détection) de l'activité ventriculaire. Ce mode permet de laisser s'exprimer une conduction auriculo-ventriculaire normale, c'est-à-dire où chaque événement auriculaire (détection auriculaire correspondant à une activité spontanée, ou stimulation auriculaire) est suivi d'une détection ventriculaire associée.

Très avantageusement, le dispositif permet un fonctionnement non seulement en mode AAI, mais également en mode DDD de stimulation double chambre.

Les dispositifs multisite implantés utilisés actuellement sont en effet très souvent des dispositifs qui comprennent des circuits de stimulation et de détection à la fois sur l'oreillette et le ventricule et peuvent opérer selon deux modes de fonctionnement, DDD ou AAI. Ces dispositifs peuvent être notamment pourvus d'un mode dénommé "DDD-CAM" assurant une commutation automatique du mode (CAM) de DDD en AAI et inversement.

Le mode de fonctionnement de base d'un stimulateur DDD/AAI est un mode AAI, maintenu tant que la conduction atrio-ventriculaire est normale (le mode AAI étant en fait un mode DDD comprenant un délai atrio-ventriculaire allongé). Dans certaines circonstances peuvent cependant apparaître des BAV entraînant un trouble temporaire de dépolarisation du ventricule. Dans ce cas, le stimulateur bascule automatiquement en mode DDD automatique, avec des paramètres optimisés pour cette situation de BAV temporaire. Après disparition du BAV, et donc rétablissement de la conduction auriculo-ventriculaire, dès lors qu'un certain nombre de conditions sont remplies le stimulateur retourne automatiquement au mode AAI. Cette commutation entre modes DDD et AAI est notamment décrite dans les EP-A-0 488 904 (ELA Médical) et EP-A-1 346 750 (ELA Médical).

Ainsi, en cas de survenue d'un BAV, et donc de commutation en mode DDD, le stimulateur se retrouvera concrètement dans le même type de fonctionnement qu'avec le mode de resynchronisation ventriculaire initial, induit par la perte de capture sur l'une des sondes ventriculaires.

En revanche, si le patient ne présente pas de trouble de la conduction (absence de BAV et délai auriculo-ventriculaire normal), le rythme ventriculaire pourra s'exprimer spontanément.

La perte de capture pouvant être dans certains cas transitoire, un test de capture est effectué à intervalles réguliers pour détecter une éventuelle réapparition de la capture perdue.

Dans ce dernier cas, c'est-à-dire s'il est possible d'obtenir à nouveau une stimulation efficace sur les deux ventricules, le dispositif peut retourner au mode de resynchronisation ventriculaire initial.

Le retour à ce mode initial peut être conditionné à l'écoulement d'un certain délai, ou au comptage d'un nombre minimum de cycles.

En variante ou en complément, le basculement du système peut être ensuite inhibé pendant une durée donnée, par exemple pendant quatre jours, de façon à éviter des commutations de mode multiples sur une courte période, qui pourraient provoquer des perturbations du rythme avec des conséquences plus graves que le simple maintien du mode de fonctionnement initial.

L'invention que l'on vient de décrire est tout à fait adaptée aux dispositifs "triple chambre", c'est-à-dire avec des sondes disposées sur les deux ventricules et sur l'oreillette droite, configuration la plus fréquemment rencontrée.

Mais elle est également applicable à une configuration où le dispositif multisite comporte des sondes sur les deux ventricules et sur l'oreillette gauche, ou encore en configuration "quadruple chambre", avec des sondes sur les deux ventricules et les deux oreillettes.

Ces diverses configurations peuvent être des configurations permanentes du dispositif ou, avantageusement, des configuration modifiables dynamiquement, comme décrit dans le EP-A-0 925 806, où le dispositif peut choisir et modifier la configuration de stimulation de manière à optimiser le traitement de l'insuffisance cardiaque.

## Revendications

1. Un dispositif médical implantable actif tel qu'un stimulateur cardiaque, défibrillateur et/ou cardioverteur du type multisite triple ou quadruple chambre, comprenant :
- des moyens de détection ventriculaire droite et gauche, aptes à détecter un potentiel de dépolarisation spontanée dans le ventricule droit et dans le ventricule gauche,
- des moyens de détection auriculaire droite, et éventuellement de détection auriculaire gauche, aptes à détecter un potentiel de dépolarisation spontanée dans l'oreillette droite.et, éventuellement, dans l'oreillette gauche,
- des moyens de stimulation ventriculaire droite et gauche, aptes à délivrer au ventricule droit et au ventricule gauche des impulsions de stimulation respectives, et
- des moyens aptes à faire fonctionner le dispositif en mode de resynchronisation des contractions ventriculaires, comprenant des moyens pour établir un délai interventriculaire ajustable entre les instants d'application des impulsions respectivement délivrées au ventricule droit et au ventricule gauche au cours d'un même cycle cardiaque,
dispositif **caractérisé en ce qu**'il comprend en outre :
- des moyens de stimulation auriculaire, aptes à délivrer des impulsions de stimulation à l'oreillette droite,
- des moyens aptes à faire fonctionner le dispositif en mode AAI avec stimulation auriculaire droite et détection ventriculaire droite,
- des moyens de détection de capture ventriculaire, pour déterminer la détection ou la perte de capture après une stimulation ventriculaire droite et après une stimulation ventriculaire gauche, et
- des moyens de commutation de mode, aptes à commander un basculement de mode, du mode de resynchronisation vers le mode AAI, en réponse à la détection d'une perte de capture après une stimulation ventriculaire droite ou après une stimulation ventriculaire gauche.

2. Le dispositif de la revendication 1, dans lequel le dispositif comprend en outre :
- des moyens d'analyse du rythme cardiaque, aptes à détecter la survenue d'un bloc auriculo-ventriculaire,
- des moyens aptes à faire fonctionner le dispositif en mode DDD de stimulation double chambre avec association auriculo-ventriculaire,
et dans lequel :
- les moyens de commutation de mode sont également aptes à commander le basculement du mode AAI vers le mode DDD en cas de détection d'un bloc auriculo-ventriculaire.

3. Le dispositif de la revendication 1, dans lequel :
- les moyens de commutation de mode sont également aptes à commander le basculement inverse du mode AAI vers le mode de resynchronisation en réponse à la détection d'un rétablissement de la capture ventriculaire droite et gauche.

4. Le dispositif de la revendication 3, dans lequel :
- les moyens de commutation de mode comprennent des moyens pour appliquer un délai prédéterminé avant ledit basculement inverse du mode AAI vers le mode de resynchronisation, après ladite détection d'un rétablissement de la capture ventriculaire droite et gauche.

5. Le dispositif de la revendication 3, dans lequel :
- les moyens de commutation de mode comprennent des moyens pour, après basculement inverse du mode AAI vers le mode de resynchronisation, inhiber tout nouveau basculement ultérieur du mode de resynchronisation vers le mode AAI pendant une durée prédéterminée.

## Claims

1. An active implantable medical device, such as a pacemaker, defibrillator and/or cardioverter of the triple or quadruple chamber multisite type, comprising:
- means for right and left ventricular detection, able to detect a potential of spontaneous depolarization in the right ventricle and the left ventricle,
- means for right atrial detection and possibly left atrial detection, able to detect a potential of spontaneous depolarization in the right atrium and possibly in the left atrium,
- means for right and left ventricular stimulation, able to deliver respective stimulation pulses to the right ventricle and to the left ventricle, and
- means able to operate the device in a mode of resynchronization of the ventricular contractions, comprising means to establish an adjustable inter-ventricular delay between the moments of application of the pulses respectively delivered to the right ventricle and to the left ventricle during the same cardiac cycle,
said device being **characterized in that** it further comprises:
- means for atrial stimulation, able to deliver stimulation pulses to the right atrium,
- means able to operate the device in AAI mode with right atrial stimulation and right ventricular detection,
- means for detection of ventricular capture, to determine the detection or the loss of capture after a right ventricular stimulation and after a left ventricular stimulation, and
- means for mode switching, able to control a mode switching from the resynchronization mode towards AAl mode in response to the detection of a loss of capture after a right ventricular stimulation or after a left ventricular stimulation.

2. The device of claim 1, wherein the device further comprises:
- means for analyzing the heart rate, able to detect the occurrence of an atrio-ventricular block,
- means able to operate the device in double-chamber stimulation DDD mode with atrio-ventricular association, and in which:
- the means for mode switching are also able to control the mode switching from AAI mode towards DDD mode in the event of detection of an atrio-ventricular block.

3. The device of claim 1, wherein:
- the means for mode switching are also able to control the opposite switching from AAI mode towards the mode of resynchronization in response to the detection of a re-establishment of the right and left ventricular capture.

4. The device of claim 3, wherein:
- the means for mode switching comprise means to apply a predetermined delay before said opposite switching from AAI mode towards the mode of resynchronization, after said detection of a re-establishment of the right and left ventricular capture.

5. The device of claim 3, wherein:
- the means for mode switching comprise means for, after opposite switching from the AAl mode towards the mode of resynchronization, inhibiting all new later switching from the mode of resynchronization towards the AAI mode for a predetermined length of time.

## Patentansprüche

1. Aktive implantierbare medizinische Vorrichtung, wie bspw. ein Herzschrittmacher, Defibrillator und/oder Kardioverter vom Dreifach- oder Vierfachraum-Multisite-Typ, mit:
- Mitteln zur Erfassung in der rechten und linken Herzkammer, die geeignet sind, ein spontanes Depolarisationspotential in der rechten Herzkammer und der linken Herzkammer zu erfassen,
- Mitteln zur Erfassung im rechten Herzvorhof, und eventuell zur Erfassung im linken Herzvorhof, die geeignet sind, ein spontanes Depolarisationspotential im rechten Herzvorhof und eventuell im linken Herzvorhof zu erfassen,
- Mitteln zur Stimulation in der rechten und linken Herzkammer, die geeignet sind, an die rechte Herzkammer und die linke Herzkammerjeweilige Stimulationsimpulse abzugeben, und
- Mitteln, die geeignet sind, die Vorrichtung im Modus der Resynchronisierung der Herzkammerkontraktionen zu betreiben, mit Mitteln zur Festlegung einer anpassbaren interventrikulären Verzögerung zwischen den Zeitpunkten der Anwendung der Impulse, die jeweils an die rechte Herzkammer und die linke Herzkammer während eine selben Herzzyklus abgegeben werden,
Vorrichtung, die **dadurch gekennzeichnet ist, dass** sie außerdem folgendes umfasst:
Mittel zur Herzvorhofstimulation, die geeignet sind, Stimulationsimpulse an den rechten Herzvorhof abzugeben,
- Mittel, die geeignet sind, die Vorrichtung im AAI-Modus zu betreiben, mit Stimulation des rechten Herzvorhofs und Erfassung an der rechten Herzkammer,
- Mittel zur Erfassung der Reizung der Herzkammer, zur Feststellung der Erfassung oder des Verlusts der Reizung nach einer Stimulation der rechten Herzkammer und nach einer Stimulation der linken Herzkammer, und
- Mittel zur Modusumschaltung, die geeignet sind, eine Modusumschaltung zu steuern, vom Resynchronisationsmodus zum AAI-Modus, in Antwort auf die Erfassung eines Reizungsverlusts nach einer Stimulation der rechten Herzkammer oder nach einer Stimulation der linken Herzkammer.

2. Vorrichtung nach Anspruch 1, bei welcher die Vorrichtung weiterhin folgendes umfasst:
- Mittel zur Analyse des Herzrhythmus, die geeignet sind, das Auftreten eines atrioventrikulären Blocks zu erfassen,
- Mittel, die geeignet sind, die Vorrichtung im DDD-Modus der Doppelkammerstimulation mit atrioventrikulärer Assoziation zu betreiben,
und bei welcher:
- die Mittel zur Modusumschaltung ebenfalls geeignet sind, den Wechsel vom AAI-Modus zum DDD-Modus im Falle der Erfassung eines atrioventrikulären Blocks zu steuern.

3. Vorrichtung nach Anspruch 1, bei welcher:
- die Mittel zur Modusumschaltung ebenfalls geeignet sind, den umgekehrten Wechsel vom AAI-Modus zum Resynchronisationsmodus in Antwort auf die Erfassung einer Wiederherstellung der Reizung der rechten und linken Herzkammer zu steuern.

4. Vorrichtung nach Anspruch 3, bei welcher:
- die Mittel zur Modusumschaltung Mittel zur Anwendung einer vorbestimmten Verzögerung vor dem umgekehrten Wechsel vom AAI-Modus zum Resynchronisationsmodus umfassen, nach der Erfassung einer Wiederherstellung der Reizung der rechten und linken Herzkammer.

5. Vorrichtung nach Anspruch 3, bei welcher:
- die Mittel zur Modusumschaltung Mittel umfassen, um nach dem umgekehrten Wechsel vom AAI-Modus zum Resynchronisationsmodus jeglichen späteren erneuten Wechsel vom Resynchronisationsmodus zum AAI-Modus während einer vorbestimmten Dauer zu unterdrücken.
